# EUROPEAN PATENT SPECIFICATION

(11) **EP 2 758 542 B1**
(45) Date of publication and mention of the grant of the patent: **23.08.2017**
(21) Application number: 12762379.1
(22) Date of filing: 19.09.2012
(51) Int. Cl.: C12Q 1/68

(54) **PROBE WITH MULTIPLE TARGET REGION SPECIFICITY AND OF TRIPARTITE CHARACTER**
SONDE MIT MULTIPLER ZIELSEQUENZSPEZIFITÄT UND VON DREITEILIGEM CHARACTER
SONDE SPECIFIQUE DE REGIONS CIBLES MULTIPLES ET AYANT UN CARACTERE TRIPARTITE

(30) Priority: 19.09.2011 GB 201116131
(43) Date of publication of application: 30.07.2014
(73) Proprietor: Epistem Limited, Manchester M13 9XX (GB)
(72) Inventor: COBB,Ben, Manchester M13 9XX (GB)
(74) Representative: Williams, Gareth Owen
(86) International application number: PCT/GB2012/052305
(87) International publication number: WO 2013/041853

(56) References cited:
- WO-A1-2005/118844
- WO-A1-2006/095941
- WO-A1-2011/087928
- WO-A2-03/054223
- WO-A2-2004/098386
- WO-A2-2011/027966
- US-A1- 2007 259 347

## Description

### FIELD OF THE INVENTION

The present invention relates to a nucleic acid probe for detecting target nucleic acid sequences, and more particularly to a probe for detecting variant nucleic acid sequences from genomic or amplified DNA. Further aspects of the invention relate to methods for detecting variant nucleic acid sequences.

### BACKGROUND TO THE INVENTION

Nucleic acid probes are often used to identify the presence of specific target sequences in genomic or amplified DNA. The annealing or melting temperature of the probe to the target is affected by the length of the complementary region shared by the probe and the target, and by the existence of any mismatches between the otherwise complementary base pairs. This can be used to detect the presence of variants, for example SNPs or multiple repeats. A probe can be designed to have a first melting temperature (Tm) to a wild type sequence, and the annealing of the probe to the target monitored, for example through development of fluorescence on annealing. If the Tm is different to the expected value, then the target sequence includes a variant.

However, this process may be difficult to implement with longer probe regions. In particular, a long probe may not provide adequate variation in Tm between a wild type and a variant sequence.

The present invention is intended to address these and other disadvantages with conventional probes.

US2007/0065847 describes a collection of nucleotide probes labelled with a common sequence to act as a tag. The probes are used for probing libraries of compounds.

WO2011/027966 describes probes having first and second regions, the first region including a reporter molecule and the second region including a quencher molecule. When the probe hybridises to a target, exonucleases can cleave the reporter from the quencher, leading to signal detection. When there is a mismatch such that the reporter portion is not hybridised, the reporter is not cleaved, and there is no signal detection.

WO2007/058499 describes probes for respiratory viruses having first and second hybridisation portions separated by a separation portion.

US2007/0259347 describes probes for screening arrays, the probes having two portions separated by a linker where the linker segment is selected to minimise homology noise associated with hybridisation.

US2003/0170711 describes oligonucleotide probes including universal bases, including 2-deoxyinosine.

WO2011/087928 describes oligonucleotide probes for detecting KRAS and PIK3CA SNPs in a background of wild type sequences, including polydeoxyinosine linkers.

WO2006/095941 describes a nucleic acid molecule using a dual specificity oligonucleotide having a 5' high Tm specificity portion, a separation portion, and a 3' low Tm specificity portion. The 3' segment does not include tandem repeat sequences.

WO2003/054223 describes tripartite molecular beacons including a first portion having a hairpin stem loop structure, and fluorophores and quenchers on second and third portions of the beacon respectively. The portions do not include tandem repeat sequences.

WO2004/098386 describes a tripartite oligonucleotide having a first hybridizable domain, a second bridging block domain, and a third binding domain. The third domain does not include tandem repeat sequences.

### SUMMARY OF THE INVENTION

According to a first aspect of the present invention, there is provided a nucleic acid probe in accordance with claim 1. The invention also provides methods of using such probes, as recited in independent claims 12 and 13. Further aspects of the invention are described in the dependent claims.

The presence of the linker region allows the probes to be split into functional elements that have different hybridisation characteristics. Inclusion of these linkers creates 'bubble' structures, isolating the elements of the probe from a thermodynamic perspective, to provide regions with different binding characteristics. Further, the presence of the linker nucleic acid sequence allows the whole probe to have the characteristics of a single polynucleotide molecule, but to behave as if composed of separate shorter nucleic acid probes. The linker region may fold to form a loop out when the first and second sequences hybridise to their respective target sequences.

In certain embodiments, the probe may be further extended to have further multiple hybridisation regions separated by multiple linkers.

This can be used in a number of different ways. For example, the probe structure allows probing of contiguous regions, where longer probes (for example, a single probe spanning both first and second target regions) would not provide adequate reporting through Tm analysis to differentiate variants.

Preferably the linker is a nucleoside linker; more preferably the linker comprises polydeoxyribonucleotides; most preferably the linker comprises or consists of polydeoxyinosine. Deoxyinosine has a low melting temperature relative to natural bases due to weaker hydrogen bonding. Other nucleosides may be used.

Preferably the linker is up to 5, 10, 15, 20, 30, 40, 50 nucleotides in length.

At least one of the first and second nucleic acid sequences is a reporter region. A reporter region includes a labelled moiety; preferably a fluorescent label. This allows detection of the probe in the event of binding to a target sequence, and monitoring of annealing over a temperature range in order to determine the presence of any variant target sequences. The probe preferably does not comprise a quencher moiety, nor is the label intended to be used with a quencher. Suitable labels include FAM, TET, HEX, ROX, TAMRA, Cy3, and Cy5. Other suitable labels will be known to the skilled person. Preferably the label is incorporated on to a T nucleotide, although any suitable nucleotide may be used.

The reporter region is preferably 15-200 nt in length, more preferably 15-150, more preferably still 15-100, or 20-100, 30-80, 40-60, or around 50 nt in length.

The reporter region is designed to have a first Tm in respect of an entirely complementary target sequence, and an altered, preferably lower, Tm, in respect of a variant target sequence.

In this embodiment, the melting temperature of the first sequence annealed to the first target nucleic acid sequence may be different to that of the second sequence annealed to the second target nucleic acid sequence. Preferably, however, both first and second nucleic acid sequences are selected to have similar Tm to their respective target sequences.

The reporter region may further comprise a blocking region; that is, a portion which serves to block extension of the nucleic acid strand by DNA polymerase, so preventing strand extension during, for example, PCR. A polymerase enzyme blocking group is one which should have the functional properties of blocking further elongation of the polymer. A blocking group may be any chemical group which can be attached to a nucleotide which will allow the 5' end of the modified nucleotide to attach to a 3' end of another nucleotide in a DNA chain but will not allow attachment of a nucleotide to the 3'hydroxyl group of the modified nucleotide. Suitably, the absence of an OH group in the 3' position will prevent further elongation by polymerase activity. In a particularly preferred embodiment, the blocking group is selected from acetyl, CH₃, glycyl, leucyl and alanyl groups. In another embodiment, the blocking group may be in the form of a di or tri peptide.

In an embodiment of the invention, both the first and second nucleic acid sequences are reporter regions. They may include different labels. Such a probe may be used as a multiplex reporter, allowing detection of target sequences over an extended range with a single probe.

In this embodiment, the probe is split into multiple discrete reporter regions. Each reporter region has different annealing temperatures and has 1 or more fluorescent nucleotides, preferably FAM-T, or different / multiple colours. The reporter may be used to report the presence of specific sequences or sequence variants, SNPs, insertions, deletions, etc. This allows multiple sequences over an extended range to be detected with a single probe. Each region is tuned to have a similar Tm in the case of the wild type target sequence, but a shifted Tm in the case of a mutation; this means that a user only has to detect the shifted Tm to know the variant is present.

As an alternative to detecting multiple target sequences, the probe may be structured for example to provide a control binding region and a test binding region, to improve the reliability of an assay.

In an alternative embodiment of the invention, the second nucleic acid sequence may be a reporter region, and the first nucleic acid sequence is an anchor region. In this embodiment, the melting temperature of the first sequence annealed to the first target nucleic acid sequence is higher than that of the second sequence annealed to the second target nucleic acid sequence. The linker sequence may form a loop-out region or may hybridise to any inter-probe nucleic acid sequence. The Tm of the anchor region to the target sequence is significantly higher (at least 0.5, 1, 1.5°C but more generally up to 5°C to 20°C higher than the Tm of the reporter region with the target sequence.

The anchor region is preferably at least 50 nt in length, more preferably at least 60, 70, 80, 90, 100, 125, 150 nt. The anchor region is preferably no more than 200 nt in length and no less than 20 nt in length.

In use, as the anchor region has a substantially higher annealing temperature it serves to anchor the probe to the correct region. The reporter region has a lower annealing temperature to the anchor region and has 1 or more fluorescent nucleotides, preferably FAM-T. The reporter is used to report the presence of a specific sequence or sequence variants.

In preferred embodiments, the probe is used to detect size/length polymorphisms e.g. STR profiling, Fragile X, etc. The anchor region is used to anneal to the downstream of the repeat region, while the reporter region is homologous to a short stretch of repeats. Since the reporter is isolated from the main anchor region, and since the fluorophores are only on the reporter region, it makes no difference if the reporter binds at different sites to cause loop outs in the amplicon - normally this would alter the Tm of the product, making measurement difficult. In certain embodiments, the anchor region may be designed to be homologous to a short stretch of the repeat region as well as a flanking region; for example, the anchor region may include up to 50 nt of repeat sequence and a longer stretch, eg 150 nt, of flanking sequence.

Also disclosed herein is a nucleic acid probe comprising a first anchor nucleic acid sequence being complementary to a first target nucleic acid sequence; a second reporter nucleic acid sequence being complementary to a second target nucleic acid sequence; and a linker nucleic acid sequence joining the first and second nucleic acid sequences; wherein the linker separates the first and second sequences such that the melting temperatures of the first and second sequences hybridised to their respective target sequences is discrete, and where the melting temperature of the anchor sequence annealed to the first target nucleic acid sequence is higher than that of the reporter sequence annealed to the second target nucleic acid sequence; and wherein each reporter sequence comprises at least one detectable label.

The reporter preferably comprises a plurality of repeated sequence units. Preferably at least 3, 4, or 5 repeated units.

The linker preferably comprises polydeoxyinosine.

The anchor may comprise one or more repeated sequence units, but preferably comprises unique sequence. The anchor is preferably from 50 - 200 nt in length.

The probe preferably further comprises a blocking sequence to prevent strand extension during PCR.

The probe may further comprise one or more additional linker and reporter sequences.

Also disclosed herein is a nucleic acid probe comprising a first reporter nucleic acid sequence being complementary to a first target nucleic acid sequence; a second reporter nucleic acid sequence being complementary to a second target nucleic acid sequence; and a linker nucleic acid sequence joining the first and second nucleic acid sequences; wherein the melting temperature of the first reporter sequence annealed to the first target nucleic acid sequence is similar or equal to that of the second reporter sequence annealed to the second target nucleic acid sequence; and wherein each reporter sequence comprises at least one detectable label.

The probe preferably further comprises a blocking sequence to prevent strand extension during PCR.

Also disclosed herein is a method for detecting one or more polymorphisms in a target nucleic acid sequence, the method comprising:
providing a probe according to an aspect of the present invention;
contacting the probe with a test nucleic acid sequence, the test sequence having a first target nucleic acid sequence and a second target nucleic acid sequence;
allowing the first nucleic acid sequence of the probe to hybridise to the first target nucleic acid sequence; and the second nucleic acid sequence of the probe to hybridise to the second target nucleic acid sequence; and
determining the Tm of the first probe sequence hybridised with the first target sequence; and determining the Tm of the second probe sequence hybridised with the second target sequence;
wherein the Tm is indicative of the presence of a wild type sequence, or of a variant sequence.

This method may also be used to improve the utility of melting temperature whereby the Tm of a first reporting region acts as a reference to measure the Tm of the variant sequence region. Generally the position of the recorded melting temperature which is useful in determining the underlying sequence, may be affected by the concentration of salts in the reaction mixture. By comparing the melting position of the first reporter region as a known sequence, it is possible to more accurately determine the sequence under the second reporter sequence since both regions will be equally affected by interfering substances such as external salts.

Also disclosed herein is a method for detecting one or more polymorphisms in a target nucleic acid sequence, the method comprising:
providing a probe according to an aspect of the present invention;
contacting the probe with a test nucleic acid sequence, the test sequence having a first target nucleic acid sequence and a second target nucleic acid sequence;
allowing the first nucleic acid sequence of the probe to hybridise to the first target nucleic acid sequence; and the second nucleic acid sequence of the probe to hybridise to the second target nucleic acid sequence; and
determining the Tm of the first probe sequence hybridised with the first target sequence; and determining the Tm of the second probe sequence hybridised with the second target sequence;
comparing the determined Tm of the first probe sequence with the expected Tm of the first probe sequence;
normalising the determined Tm of the second probe sequence based on the difference of the determined and expected Tm of the first probe sequence;
wherein the normalised Tm of the second probe sequence is indicative of the presence of a wild type sequence, or of a variant sequence.

Also disclosed herein is a method for detecting tandem repeats in a target nucleic acid sequence, the method comprising:
providing a probe according to an aspect of the present invention, wherein the reporter sequence comprises a number of tandem repeat units;
contacting the probe with a test nucleic acid sequence, the test sequence having a first target nucleic acid sequence and a second target nucleic acid sequence, wherein the first target sequence is complementary to the probe anchor sequence, and the second target sequence comprises a number of tandem repeats complementary to the tandem repeat units of the reporter sequence;
allowing the anchor nucleic acid sequence of the probe to hybridise to the first target nucleic acid sequence; and the second nucleic acid sequence of the probe to hybridise to the second target nucleic acid sequence; and
detecting binding or otherwise of the reporter sequence to the test nucleic acid sequence.

Binding may be detected by determining the Tm of the reporter sequence to the test nucleic acid sequence, and/or by detecting label (eg, fluorescence) from the reporter sequence. Binding indicates that the number of repeats in the target sequence is greater than or equal to the number of repeats in the reporter sequence; if there are more repeats in the reporter sequence, then there is either no binding or reduced binding. In certain embodiments, the Tm may be considered to be generally proportional to the number of repeats in the target sequence, and the Tm may be determined in order to determine the number of repeats. Alternatively, or in addition, the reporter sequence of the probe may include one or more labelled moieties in each repeat unit; in this way, the detected label may be taken as being proportional to the number of repeats in the target sequence.

### BRIEF DESCRIPTION OF THE DRAWINGS

These and other aspects of the present invention will now be described by way of example only and with reference to the accompanying drawings, in which:
Figure 1 shows a first probe architecture;
Figures 2 to 5 show alternative duplex structures formed through binding of the probe of figure 1 to a target sequence;
Figure 6 shows a second probe architecture;
Figure 7 shows common mutant codons in the M. tuberculosis rpoB gene;
Figure 8 shows the sequence of a portion of the M. tuberculosis rpoB gene, together with the sequences of certain mutant genes and the sequence of a probe for detecting the presence of such mutants;
Figure 9 shows alternative duplex structures formed through binding of the probe of Figure 8 to target sequences;
Figure 10 shows example melt curves obtained from the duplexes of Figure 9;
Figure 11 shows a sequence alignment of a portion of the vaccinia genome with that of other pox viruses;
Figure 12 shows a probe for detection of the pox viruses;
Figure 13 compares sensitivity of the linker probe of Figure 12 with that of a conventional vaccinia probe;
Figure 14 shows melt curve analysis of the linker probe of Figure 12 and a conventional probe with various amplified pox virus genomic sequences;
Figure 15 shows primer sequences and amplification conditions for amplifying the pox virus fragment used in Figure 14; and
Figure 16 shows primer and probe sequences, and melt curve analysis, for a short tandem repeat in the human tyrosine hydroxylase gene.

### DETAILED DESCRIPTION OF THE DRAWINGS

Disclosed herein is a nucleic acid probe which is split into two or more functional elements, separated by a nucleic acid linker. The linker is preferably deoxyinosine, which has a low Tm relative to the naturally occurring DNA bases. Other suitable linkers may be used.

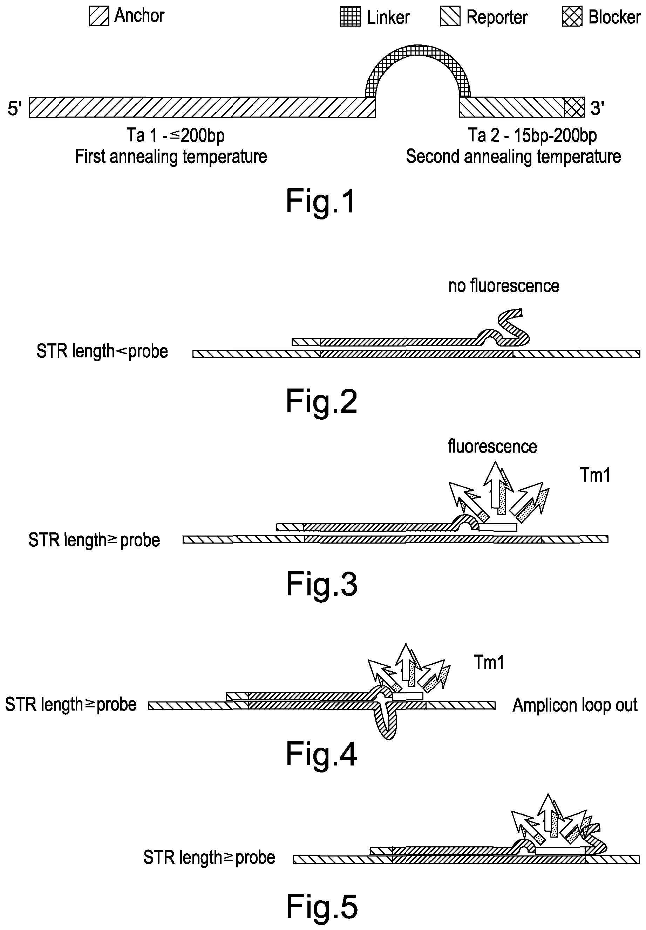

Figure 1 shows schematically a first embodiment of a probe. The probe includes an anchor region, of around 100 - 200 nt in length, separated by a deoxyinosine linker of 5 nt in length from a reporter region, of from 15 - 200 nt in length. The anchor region is designed to be complementary to a first target region which is downstream from a repeated sequence region in a target nucleic acid. The reporter region is complementary to the repeated sequence region, and includes a known number of repeat units. The reporter region also includes one or more labelled nucleotides, preferably FAM-T. In certain embodiments, each repeat unit includes a labelled nucleotide.

The probe sequences are designed such that the Tm of the anchor region to the target is significantly higher than the Tm of the reporter to the target, which in turn is significantly higher than the Tm of the linker to any of the sequences in the target nucleic acid.

Figures 2 to 5 show various different scenarios for the binding of the probe to a target. In these scenarios, the probe is being used to determine size/length polymorphisms e.g. STR profiling, Fragile X, etc. The anchor region is used to anneal the probe to the downstream of the repeat region, while the reporter region is homologous to a short stretch of repeats. Since the reporter is isolated from the main anchor region, and since the fluorophores are only on the reporter region, it makes no difference if the reporter binds at different sites to cause loop outs in the amplicon - normally this would alter the Tm of the product, making measurement difficult.

Where the length of the target repeated sequence region is less than that of the reporter region (Figure 2), then the reporter region does not bind to the target, and there is no fluorescence from the label, and no melt curve obtained by measuring Tm of the reporter. However, as the anchor region has bound to the target, this can be measured (eg, by determining Tm), in order to confirm that the probe does bind and that the repeated sequence region is absent.

Where the length of the target repeated sequence region is equal to or longer than the length of the reporter region, then any of the scenarios in Figures 3 to 5 may occur. In Figure 3, the reporter is kept small to 1, 2, 3, 4 or 5 repeat lengths which limits the impact of any loop-out effects on the Tm of the reporter:amplicon duplex. Examples of use are for large repeat structures such as Fragile X where it is important to 'band' the number of repeats; e.g. < 50, > 100, > 200. Alternatively, a small loop-out may occur (Figure 4); again, since the reporter is small, there is minimal effect on the Tm of the reporter:amplicon duplex, and the presence of at least the number of repeats on the probe can be determined.

If the reporter length is increased to accommodate multiple repeats (Figure 5), then the Tm of the reporter amplicon duplex will be proportional to the number of repeats. Unbound portions of the reporter will not fluoresce due to self-quenching of the label. One or two nucleotides of each repeat in the reporter are labeled. This methodology may be important in STR analysis where you are interested in the number of 3, 4 or 5bp repeats up to 15x or 20x.

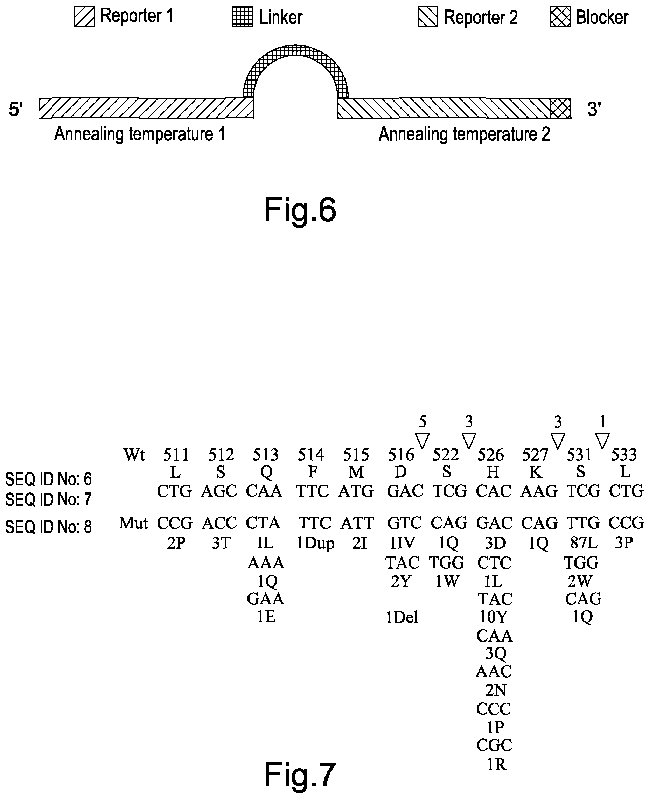

An alternative probe architecture is shown in Figure 6. This probe is a multiplexed reporter, and includes two separate reporter regions joined by a linker. Each reporter region has different annealing temperatures and has 1 or more fluorescent nucleotides, preferably FAM-T, or different / multiple colours. The reporter is used to report the presence of a specific sequence or sequence variants (eg, SNPs, insertions, deletions, etc). This allows multiple sequences over an extended range to be detected with a single probe. Each region is tuned to have a similar - but not identical - Tm in the case of the wild type sequence, but a shifted Tm in the case of a mutation so that a user only has to detect the shifted Tm to know the variant is present. By "similar" is meant that the Tm differs by at most 2, 1.5, 1, 0.5 deg C.

The probe could also be structured for example to provide a control binding region and test binding region.

An example of use of a multiplexed reporter probe to detect variants in the Mycobacterium tuberculosis rpoB gene is now given. Multi drug resistance in M. tuberculosis is complex. Rifampin is a first line M. tuberculosis medication and is the main target to identify in the field prior to treatment. Rifampin resistant M. tuberculosis have mutations in the 81-bp core region of the rpoB gene, which encodes the β-subunit of RNA polymerase. 96% of Rifampin resistant clinical isolates of M. tuberculosis have mutations in this gene. Mutations in codons 516, 526, or 531 result in high level Rifampin resistance. However, detecting mutations across an 81-bp gene region would typically require multiple conventional probes, several of which would need to overlap, so requiring multiple detection steps.

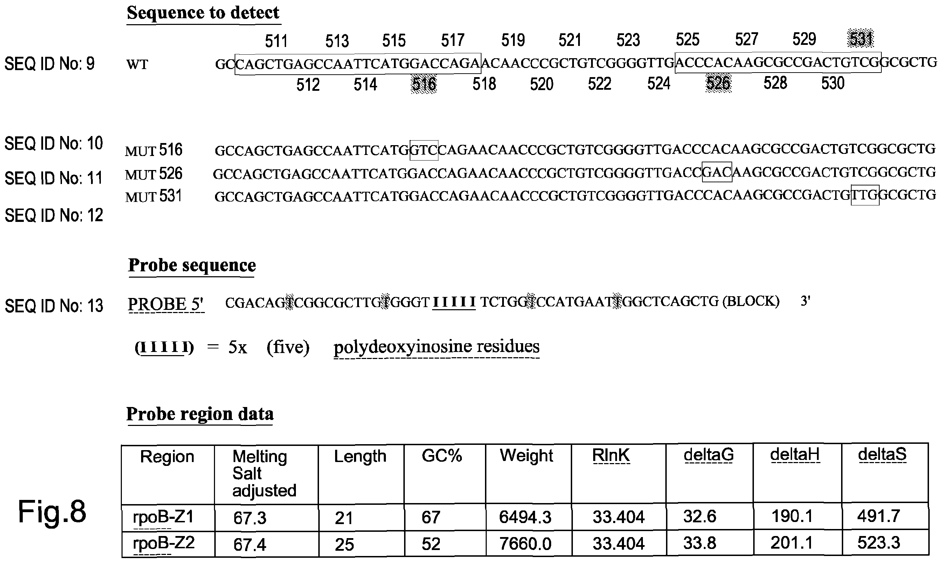

Figure 7 shows wild type and variant codons in a section of the rpoB gene; note that 9 codons are shown from a 21 codon (63 nt) region. The present inventors have designed a probe (shown in Figure 8, labeled PROBE) to detect and report the presence of the any of the potential M. tuberculosis genotypes with mutations in the main resistance codons 516, 526, or 531.

Figure 8 also shows the wild type (WT) sequence of the rpoB gene, together with three mutant sequences (MUT516, MUT526, and MUT531). The boxed regions of the WT sequence are those to which the probe is designed to hybridise. The probe itself includes a first reporter sequence spanning codons 531 to 525, including potential mutant codons 526 and 531, a linker comprising five polydeoxyinosine residues (indicated as "I"), and a second reporter sequence spanning codons 518 to 510, including potential mutant codon 516. Each of the reporter sequences includes two labeled T residues (highlighted). The second reporter sequence is also adjacent a PCR blocker moiety.

The probe binding to WT sequence in both positions gives the same Tm of 60C for each reporter sequence. The presence of mutant sequences shifts this to 52C-56C.

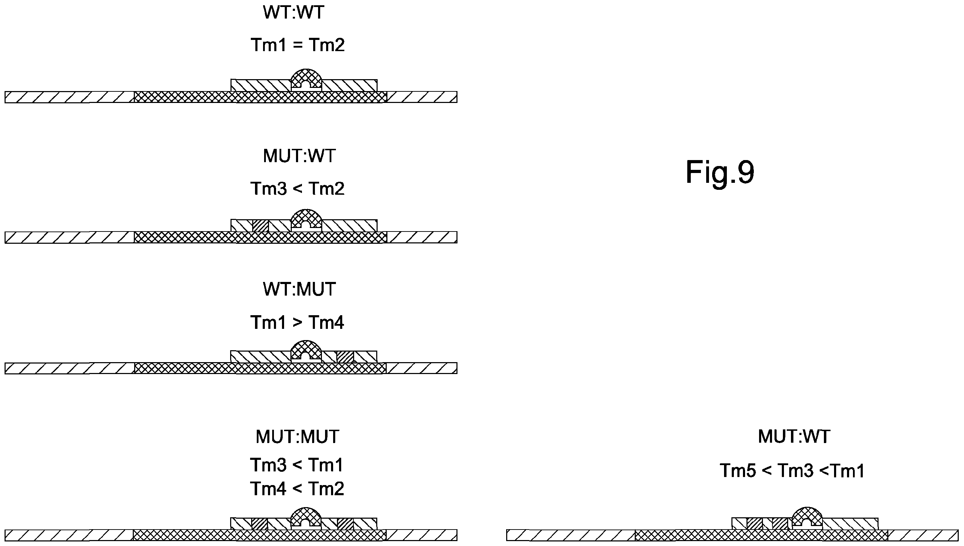

Figure 9 shows the possible binding patterns of the probe to target sequence. Where both reporter sequences bind to the wild type (WT:WT), the Tm of the first reporter (Tm1) equals the Tm of the second reporter (Tm2). A single mutant in the first reporter (MUT:WT) lowers the Tm of the first reporter to Tm3, which is lower than Tm2. A single mutant in the second reporter (WT:MUT) lowers the Tm of the second reporter to Tm4, which is lower than Tm1. A single mutant in each reporter (MUT:MUT) shifts the respective Tms to Tm3 and Tm4. Finally, the presence of a second mutant in the first reporter target (MUT:WT) lowers the Tm to Tm5, which is lower than Tm3.

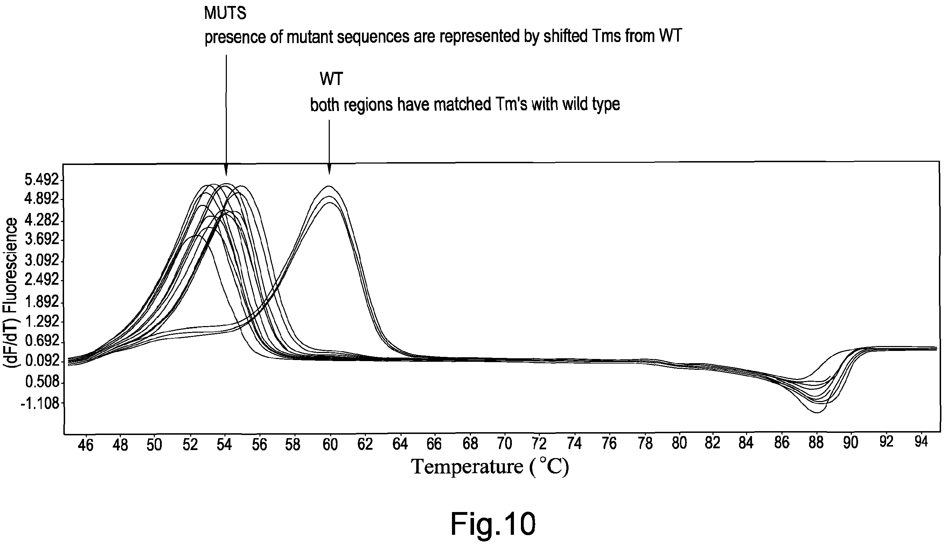

Each of these Tms is distinguishable from one another, and so the single probe may be used to determine the presence of three distinct mutations in the rpoB gene. Figure 10 gives representative melt curves from such experiments. The presence of wild type of mutant sequences can be clearly distinguished. Thus, this probe architecture permits simple multiplex detection of multiple mutations across a relatively long stretch of genome.

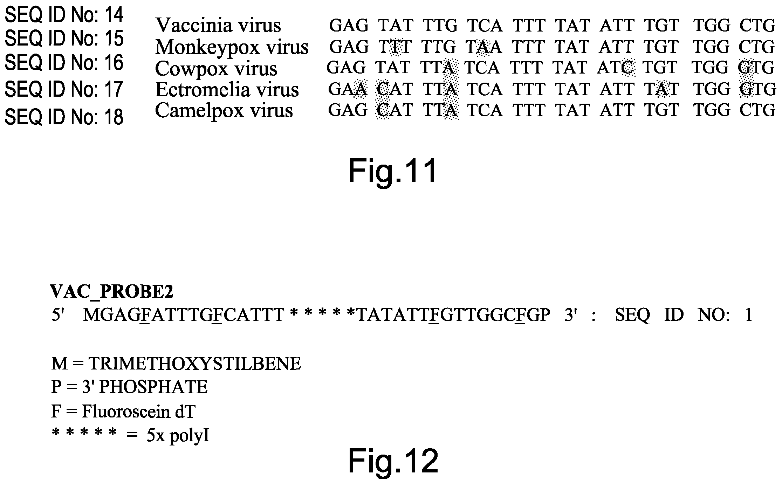

A further illustrative use of such a probe is shown in Figures 11 to 15, which illustrate use of a probe for detection of the Vaccinia virus, and distinguishing this virus from related pox viruses.

Figure 11 compares a 30 nucleotide portion of the genomic sequence of Vaccinia with that of the monkeypox, cowpox, ectromelia, and camelpox viruses. It will be seen that there are eight bases which may differ across this sequence.

Figure 12 illustrates a probe for detection of this region of the virus genome and distinguishing the various viruses from one another. The probe includes a first reporter region separated from a second reporter region by a sequence of five inosine residues. The first reporter region includes a trimethoxystilbene cap. Each reporter region includes two fluorescently labelled T residues.

The probe sequence is:
5'-GAG T*AT TTG T*CA TTT IIIII TAT ATT T*GT TGG CT*G-3' : SEQ ID NO: 1 Capped with a 5' trimethoxystilbene, and a 3' phosphate. I = inosine. T* = fluoresceine labelled T.

The two reporter regions span the 30 nucleotide sequence shown in Figure 11, and both cover a number of potential variation sites.

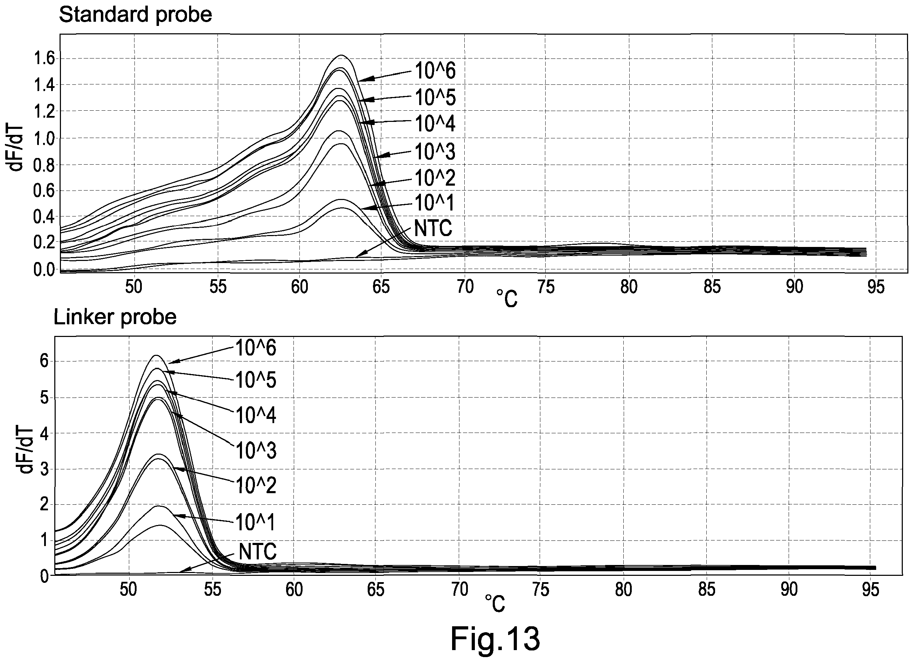

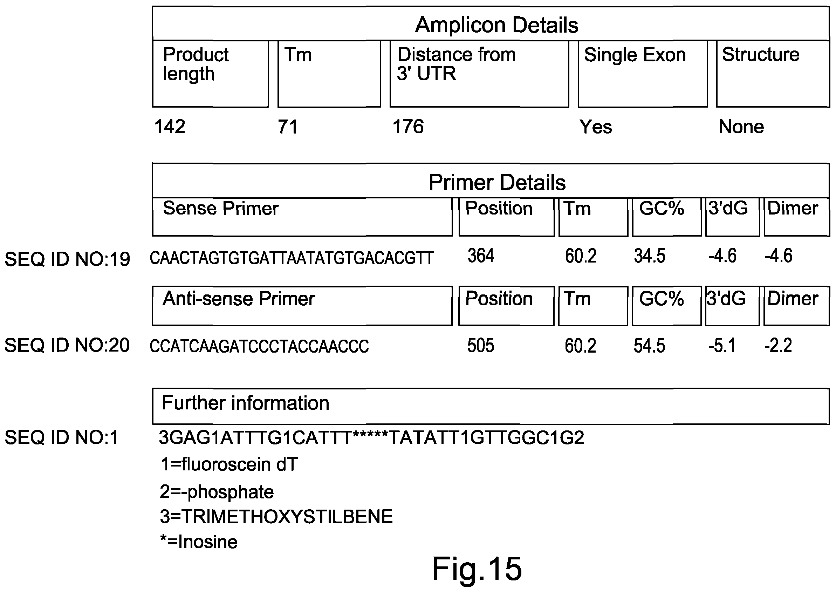

Figure 13 compares the sensitivity of the probe with that of a standard vaccinia probe spanning the same 30 nt sequence. The linker probe is significantly more sensitive at the same copy number. To compare the sensitivity, a portion of the genome is amplified using the primers and conditions indicated in Figure 15, and then a melt curve assay conducted using the relevant probe on the amplicon.

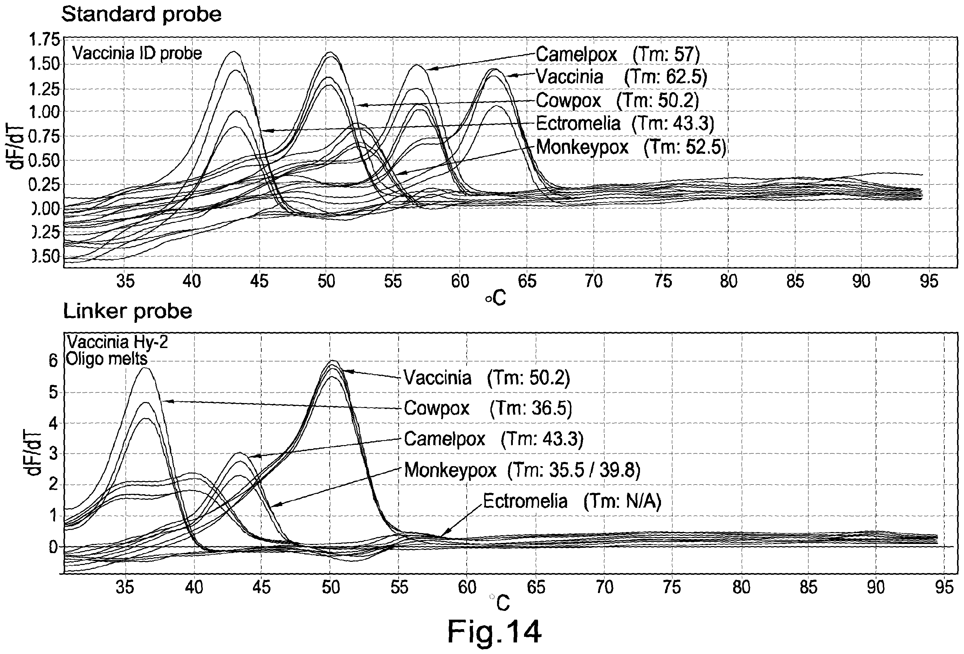

Figure 14 shows melt curves for the vaccinia probe ("linker probe") and a conventional vaccinia probe ("standard probe") when hybridised to different pox virus target sequences. The range of Tm spanned by the linker probe is much smaller, but the linker probe provides improved discrimination between the various pox viruses, and allows the five viruses to be clearly identified and distinguished using a single probe. The use of a linker probe compared with a conventional probe lowers the Tm of the product, and lowers the Tm range to enable multiplexing higher in the range. The probes of the invention allow up to five base mismatches over a short region of 30 bases to be identified, so allowing identification of the pox viruses.

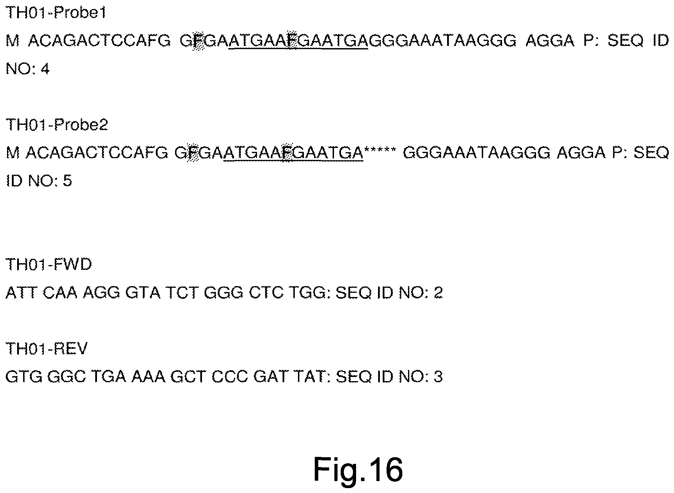

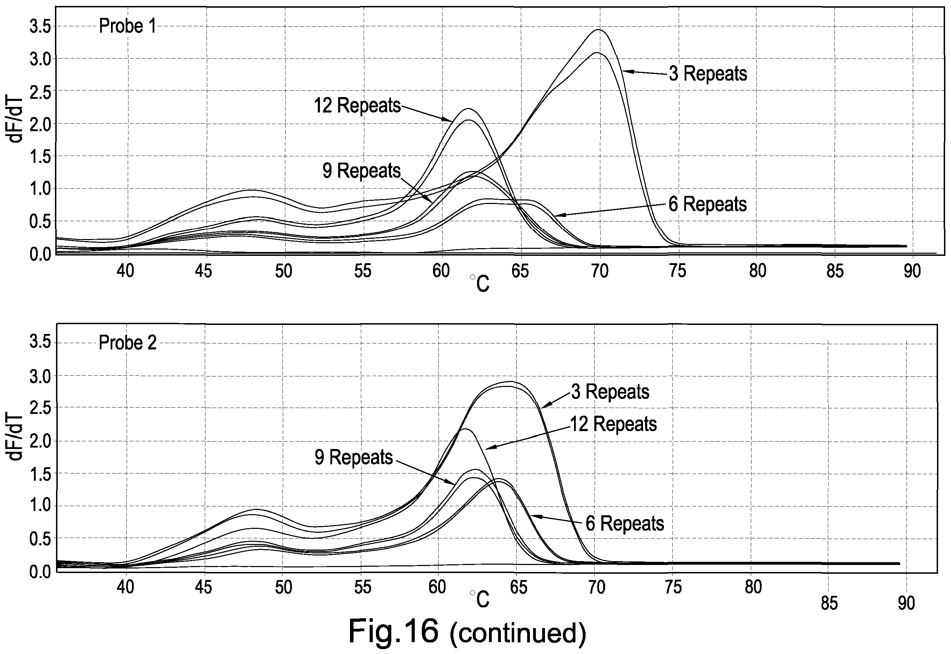

Figure 16 shows primer and probe sequences for analysing a short tandem repeat (STR) in the human tyrosine hydroxylase gene.

The forward and reverse primers are:
TH01-FWD
   ATT CAA AGG GTA TCT GGG CTC TGG : SEQ ID NO: 2
TH01-REV
   GTG GGC TGA AAA GCT CCC GAT TAT: SEQ ID NO: 3

The TH gene includes a short tandem repeat sequence of (AATG)n, which is known to be polymorphic. To confirm that the present invention is capable of distinguishing between different lengths of STR, two probes were compared for senstivity and accuracy. Both probes were of the same sequence, probe 1 was prepared without a linker, while probe 2 includes a five inosine base sequence acting as a linker between the two portions of the probe. Probe sequences were: 
TH01-Probe1
M ACAGACTCCAFG GFGAATGAAFGAATGAGGGAAATAAGGG AGGA P: SEQ ID
NO: 4
 
TH01-Probe2
M ACAGACTCCAFG GFGAATGAAFGAATGA***** GGGAAATAAGGG AGGA P: SEQ
ID NO: 5
 (M = trimethoxystilbene; P = phosphate; * = inosine; F = fluoresceine labelled T).

After amplifying the target sequence with the primers, melt curve analysis was performed with the amplicon and either probe 1 or probe 2. The curves are shown in Figure 16. Although both probes are capable of distinguishing between 3, 6, 9, and 12 copies of the repeat, probe 2, with the linker, provides discrimination between different numbers of repeats over a much narrower temperature range.

## Claims

1. A nucleic acid probe comprising a first anchor nucleic acid sequence being complementary to a first target nucleic acid sequence; a second reporting nucleic acid sequence being complementary to a second target nucleic acid sequence; and a linker nucleic acid sequence joining the first and second nucleic acid sequences; wherein the linker separates the two first and second sequences such that the melting temperature of the anchor sequence annealed to the first target nucleic acid sequence and of the reporter sequence annealed to the second target nucleic acid sequence are discrete and wherein the melting temperature of the anchor sequence annealed to the first target nucleic acid sequence is higher than that of the second sequence annealed to the second target nucleic acid sequence and the reporter sequence comprises tandem repeat sequences; wherein the tandem repeat comprises at least 3 identical copies of a sequence in tandem and wherein the probe does not comprise a quencher moiety.

2. The probe of claim 1 wherein the linker comprises a polydeoxyribonucleotide, and/or wherein the linker comprises or consists of polydeoxyinosine.

3. The probe of any preceding claim wherein the linker is up to 5, 10, 15, 20, 30 40, 50, nucleotides in length.

4. The probe of any preceding claim wherein at least one of the first and second nucleic acid sequences is a reporter region including a labelled moiety; preferably a fluorescent label; preferably wherein the reporter region is 15-200 nt in length, more preferably 15-150, more preferably still 15-100, or 20-100, 30-80, 40-60, or around 50 nt in length; and/or wherein the reporter region is designed to have a first Tm in respect of an entirely complementary target sequence, and an altered, preferably lower, Tm, in respect of a variant target sequence.

5. The probe of any preceding claim further comprising a blocking region which serves to block extension of the nucleic acid strands by DNA polymerase.

6. The probe of any preceding claim wherein both the first and second nucleic acid sequences are reporter regions; preferably wherein both reporter regions are selected to have a similar Tm in the case to binding to the wild type target sequence, and reduced Tms in the case of binding a mutant target sequence; preferably the reduced Tm for the first reporter region differs from that of the second reporter region.

7. The probe of any claims 1 to 5 wherein the Tm of the anchor region : target sequence duplex is significantly higher (at least 0.5, 1, 1.5, but more generally up to 5 to 20 deg C higher) than the Tm of the reporter region to the target sequence; and/or wherein the anchor region is at least 50 nt in length, more preferably at least 60, 70, 80, 90, 100, 125, 150 nt.

8. The probe of claim 7 wherein the anchor region is no more than 200 nt in length; and
optionally wherein the anchor region comprises tandem repeat sequences and unique sequences.

9. The probe of any of claims 1 to 6 wherein the first and second target nucleic acid sequences are found in the M tuberculosis genome; or wherein the first and second target nucleic acid sequences are found in the vaccinia pox virus genome.

10. The probe of any preceding claim wherein the first and second target sequences are within 200, 150, 100, 50, 40, 30, 20, 15, 10, 5, 4, 3, 2, 1 nucleotides of one another in a genomic sequence.

11. A nucleic acid probe according to any preceding claim further comprising one or more further linker sequences; and one or more further nucleic acid sequences for hybridisation to one or more further target sequences.

12. A method for detecting tandem repeats in a target nucleic acid sequence, the method comprising:
providing a probe according to any preceding claim, the probe comprising a reporter sequence having a number of tandem repeat units;
contacting the probe with a test nucleic acid sequence, the test sequence having a first target nucleic acid sequence and a second target nucleic acid sequence, wherein the first target sequence is complementary to the probe anchor sequence, and the second target sequence comprises a number of tandem repeats complementary to the tandem repeat units of the reporter sequence;
allowing the anchor nucleic acid sequence of the probe to hybridise to the first target nucleic acid sequence; and the second nucleic acid sequence of the probe to hybridise to the second target nucleic acid sequence; and
detecting binding or otherwise of the reporter sequence to the test nucleic acid sequence.

13. A method for detecting one or more polymorphisms in a target nucleic acid sequence, the method comprising:
providing a probe according to any one of claims 1 to 11;
contacting the probe with a test nucleic acid sequence, the test sequence having a first target nucleic acid sequence and a second target nucleic acid sequence;
allowing the first nucleic acid sequence of the probe to hybridise to the first target nucleic acid sequence; and the second nucleic acid sequence of the probe to hybridise to the second target nucleic acid sequence; and
determining the Tm of the first probe sequence hybridised with the first target sequence; and
determining the Tm of the second probe sequence hybridised with the second target sequence;
comparing the determined Tm of the first probe sequence with the expected Tm of the first probe sequence;
normalising the determined Tm of the second probe sequence based on the difference of the determined and expected Tm of the first probe sequence;
wherein the normalised Tm of the second probe sequence is indicative of the presence of a wild type sequence, or of a variant sequence.

## Patentansprüche

1. Nucleinsäuresonde, umfassend eine erste Anker-Nucleinsäuresequenz, die komplementär zu einer ersten Target-Nucleinsäuresequenz ist; eine zweite Reporter-Nucleinsäuresequenz, die komplementär zu einer zweiten Target-Nucleinsäuresequenz ist; und eine Linker-Nucleinsäuresequenz, die die erste und die zweite Nucleinsäuresequenz verbindet, wobei der Linker die beiden ersten und zweiten Sequenzen derart trennt, dass die Schmelztemperatur der Ankersequenz, die an die erste Target-Nucleinsäuresequenz angelagert ist, und die der Reportersequenz, die an die zweite Target-Nucleinsäuresequenz angelagert ist, diskret sind, und wobei die Schmelztemperatur der Ankersequenz, die an die erste Target-Nucleinsäuresequenz angelagert ist, höher ist als die der zweiten Sequenz, die an die zweite Target-Nucleinsäuresequenz angelagert ist, und die Reportersequenz Tandem-Wiederholungssequenzen umfasst; wobei die Tandemwiederholung mindestens drei identische Kopien einer Sequenz im Tandem umfasst, und wobei die Sonde keinen Quencher-Anteil aufweist.

2. Sonde nach Anspruch 1, wobei der Linker ein Polydesoxyribonucleotid umfasst und/oder wobei der Linker Polydesoxyinosin umfasst oder daraus besteht.

3. Sonde nach einem der vorhergehenden Ansprüche, wobei der Linker eine Länge von bis zu 5, 10, 15, 20, 30, 40, 50 Nucleotiden hat.

4. Sonde nach einem der vorhergehenden Ansprüche, wobei mindestens eine der ersten und zweiten Nucleinsäuresequenz ein Reporterbereich ist, der einen markierten Anteil einschließt; vorzugsweise eine Fluoreszenzmarkierung; wobei der Reporterbereich vorzugsweise eine Länge von 15 bis 200 nt hat, mehr bevorzugt 15 bis 150, noch mehr bevorzugt 15 bis 100 oder 20 bis 100, 30 bis 80, 40 bis 60 oder rund 50 nt hat; und/oder wobei der Reporterbereich dazu ausgebildet ist, in Bezug auf eine vollständig komplementäre Target-Sequenz über eine erste Tm zu verfügen und in Bezug auf eine variante Target-Sequenz über eine veränderte und bevorzugt niedrigere Tm zu verfügen.

5. Sonde nach einem der vorhergehenden Ansprüche, ferner umfassend einen blockierenden Bereich, der dazu dient, die Verlängerung der Stränge der Nucleinsäure durch DNA-Polymerase zu blockieren.

6. Sonde nach einem der vorhergehenden Ansprüche, wobei sowohl die erste als auch die zweite Nucleinsäuresequenz Reporterbereiche sind; vorzugsweise wobei beide Reporterbereiche im Falle eines Bindens an die Target-Sequenz vom Wildtyp so ausgewählt sind, dass sie eine ähnliche Tm haben, und im Falle des Bindens an eine mutante Target-Sequenz eine verringerte Tm haben, wobei sich vorzugsweise die verringerte Tm für den ersten Reporterbereich von der des zweiten Reporterbereichs unterscheidet.

7. Sonde nach einem der Ansprüche 1 bis 5, wobei die Tm des Ankerbereichs : Target-Sequenz-Duplex signifikant höher ist (mindestens 0,5, 1, 1,5, in der Regel jedoch bis zu 5° bis 20° C höher) als die Tm des Reporterbereichs zu der Target-Sequenz; und/oder wobei der Ankerbereich ein Länge von mindestens 50 nt hat, mehr bevorzugt mindestens 60, 70, 80, 90, 100, 125, 150 nt.

8. Sonde nach Anspruch 7, wobei der Ankerbereich eine Länge von nicht mehr als 200 nt hat; und
wahlweise wobei der Ankerbereich Tandem-Wiederholungssequenzen und nur einmal vorhandene Sequenzen umfasst.

9. Sonde nach einem der Ansprüche 1 bis 6, wobei die erste und die zweite Target-Nucleinsäuresequenz im Genom von M. tuberculosis gefunden werden; oder wobei die erste und zweite Target-Nucleinsäuresequenz im Genom des Pockenvirus von Vaccinia Virus gefunden werden.

10. Sonde nach einem der vorhergehenden Ansprüche, wobei sich die erste und die zweite Target-Sequenz innerhalb von 200, 150, 100, 50, 40, 30, 20, 15, 10, 5, 4, 3, 2, 1 Nucleotiden zueinander in einer genomischen Sequenz befinden.

11. Nucleinsäure-Sonde nach einem der vorhergehenden Ansprüche, ferner umfassend eine oder mehrere weitere Linkersequenzen; und eine oder mehrere weitere Nucleinsäuresequenzen zur Hybridisierung an einer oder mehreren weiteren Target-Sequenzen.

12. Verfahren zum Detektieren von Tandemwiederholungen in einer Target-Nucleinsäuresequenz, wobei das Verfahren umfasst:
Bereitstellen einer Sonde nach einem der vorhergehenden Ansprüche, wobei die Sonde eine Reportersequenz mit einer Anzahl von Tandem-Wiederholungseinheiten umfasst;
Kontaktieren der Sonde mit einer Test-Nucleinsäuresequenz, wobei die Testsequenz eine erste Target-Nucleinsäuresequenz und eine zweite Target-Nucleinsäuresequenz hat, wobei die erste Target-Sequenz zu der Sonden-Ankersequenz komplementär ist, und die zweite Target-Sequenz eine Anzahl von Tandem-Wiederholungen umfasst, die zu den Einheiten der Tandem-Wiederholungen der Reportersequenz komplementär sind;
die Anker-Nucleinsäuresequenz der Sonde mit der ersten Target-Nucleinsäuresequenz hybridisieren lassen; und die zweite Nucleinsäuresequenz der Sonde mit der zweiten Target-Nucleinsäuresequenz hybridisieren lassen; und
Detektieren, ob die Reportersequenz an die Test-Nucleinsäuresequenz bindet oder nicht.

13. Verfahren zum Detektieren eines oder mehrerer Polymorphismen in einer Target-Nucleinsäuresequenz, wobei das Verfahren umfasst:
Bereitstellen einer Sonde nach einem der Ansprüche 1 bis 11;
Kontaktieren der Sonde mit einer Test-Nucleinsäuresequenz, wobei die Testsequenz eine erste Target-Nucleinsäuresequenz und eine zweite Target-Nucleinsäuresequenz hat;
die erste Nucleinsäuresequenz der Sonde mit der ersten Target-Nucleinsäuresequenz hybridisieren lassen; und die zweite Nucleinsäuresequenz der Sonde mit der zweiten Target-Nucleinsäuresequenz hybridisieren lassen; und
Bestimmen der Tm der ersten Sondensequenz, die mit der ersten Target-Sequenz hybridisiert ist; und
Bestimmen der Tm der zweiten Sondensequenz, die mit der zweiten Target-Sequenz hybridisiert ist;
Vergleichen der ermittelten Tm der ersten Sondensequenz mit der erwarteten Tm der ersten Sondensequenz;
Normieren der ermittelten Tm der zweiten Sondensequenz basierend auf der Differenz der ermittelten und erwarteten Tm der ersten Sondensequenz;
wobei die normierte Tm der zweiten Sondensequenz für das Vorhandensein einer Wildtypsequenz oder einer varianten Sequenz kennzeichnend ist.

## Revendications

1. Sonde d'acides nucléiques comprenant une première séquence d'acides nucléiques d'ancrage qui est complémentaire par rapport à une première séquence d'acides nucléiques cible ; une seconde séquence d'acides nucléiques rapporteuse qui est complémentaire par rapport à une seconde séquence d'acides nucléiques cible ; et une séquence d'acides nucléiques de coupleur qui joint les première et seconde séquences d'acides nucléiques ; dans laquelle le coupleur sépare les deux première et seconde séquences de telle sorte que la température de fusion de la séquence d'ancrage qui est fusionnée sur la première séquence d'acides nucléiques cible et de la séquence rapporteuse qui est fusionnée sur la seconde séquence d'acides nucléiques cible soient discrètes, et dans laquelle la température de fusion de la séquence d'ancrage qui est fusionnée sur la première séquence d'acides nucléiques cible est supérieure à celle de la seconde séquence qui est fusionnée sur la seconde séquence d'acides nucléiques cible, et la séquence rapporteuse comprend des séquences de répétition en tandem ; dans laquelle la répétition en tandem comprend au moins 3 copies identiques d'une séquence en tandem et dans laquelle la sonde ne comprend pas une fraction d'extinction.

2. Sonde selon la revendication 1, dans laquelle le coupleur comprend un polydésoxyribonucléotide et/ou dans laquelle le coupleur comprend ou est constitué par de la polydésoxyinosine.

3. Sonde selon l'une quelconque des revendications précédentes, dans laquelle le coupleur présente une longueur qui va jusqu'à 5, 10, 15, 20, 30, 40, 50 nucléotides.

4. Sonde selon l'une quelconque des revendications précédentes, dans laquelle au moins l'une des première et seconde séquences d'acides nucléiques est une région rapporteuse qui inclut une fraction étiquetée ; de préférence une étiquette fluorescente ; de préférence, dans laquelle la région rapporteuse présente une longueur de 15 à 200 nt, de façon davantage préférable, de 15 à 150 nt, de façon encore davantage préférable, de 15 à 100 ou de 20 à 100, de 30 à 80, de 40 à 60 ou d'environ 50 nt ; et/ou dans laquelle la région rapporteuse est conçue de manière à présenter une première Tm par rapport à une séquence cible totalement complémentaire, et une Tm altérée, de préférence plus faible, par rapport à une séquence cible variante.

5. Sonde selon l'une quelconque des revendications précédentes, comprenant en outre une région de blocage qui sert à bloquer l'extension des brins d'acide nucléique par ADN polymérase.

6. Sonde selon l'une quelconque des revendications précédentes, dans laquelle les première et seconde séquences d'acides nucléiques sont toutes deux des régions rapporteuses ; de préférence, dans laquelle les deux régions rapporteuses sont sélectionnées de manière à présenter une Tm similaire dans le cas d'une liaison sur la séquence cible du type sauvage et une Tm réduite dans le cas d'une liaison d'une séquence cible mutante ; de préférence la Tm réduite pour la première région rapporteuse diffère de celle de la seconde région rapporteuse.

7. Sonde selon l'une quelconque des revendications 1 à 5, dans laquelle la Tm de la région d'ancrage par rapport au duplex de séquence cible est significativement plus élevée (elle est plus élevée d'au moins 0,5, 1, 1,5, et de façon davantage générale, de jusqu'à 5 à 20° C) que la Tm de la région rapporteuse par rapport à la séquence cible ; et/ou dans laquelle la région d'ancrage présente une longueur d'au moins 50 nt, de façon davantage préférable, d'au moins 60, 70, 80, 90, 100, 125, 150 nt.

8. Sonde selon la revendication 7, dans laquelle la région d'ancrage présente une longueur non supérieure à 200 nt et,
en option, dans laquelle la région d'ancrage comprend des séquences de répétition en tandem et des séquences uniques.

9. Sonde selon l'une quelconque des revendications 1 à 6, dans laquelle les première et seconde séquences d'acides nucléiques cibles sont trouvées dans le génome de M tuberculosis ; ou dans laquelle les première et seconde séquences d'acides nucléiques cibles sont trouvées dans le génome de poxvirus de la vaccine.

10. Sonde selon l'une quelconque des revendications précédentes, dans laquelle les première et seconde séquences cibles sont à l'intérieur de 200, 150, 100, 50, 40, 30, 20, 15, 10, 5, 4, 3, 2, 1 nucléotide(s) l'une par rapport à l'autre dans une séquence génomique.

11. Sonde d'acides nucléiques selon l'une quelconque des revendications précédentes, comprenant en outre une ou plusieurs autre(s) séquence(s) de coupleur ; et une ou plusieurs autre(s) séquence(s) d'acides nucléiques pour une hybridation sur une ou plusieurs autre(s) séquence(s) cible(s).

12. Procédé pour détecter des répétitions en tandem dans une séquence d'acides nucléiques cible, le procédé comprenant :
la fourniture d'une sonde selon l'une quelconque des revendications précédentes, la sonde comprenant une séquence rapporteuse qui comporte un certain nombre d'unités de répétition en tandem ;
la mise en contact de la sonde avec une séquence d'acides nucléiques de test, la séquence de test comportant une première séquence d'acides nucléiques cible et une seconde séquence d'acides nucléiques cible, dans lequel la première séquence cible est complémentaire par rapport à la séquence d'ancrage de sonde, et la seconde séquence cible comprend un certain nombre de répétitions en tandem qui sont complémentaires par rapport aux unités de répétition en tandem de la séquence rapporteuse ;
le fait de permettre que la séquence d'acides nucléiques d'ancrage de la sonde réalise une hybridation sur la première séquence d'acides nucléiques cible ; et que la seconde séquence d'acides nucléiques de la sonde réalise une hybridation sur la seconde séquence d'acides nucléiques cible ; et
la détection de la liaison ou autre de la séquence rapporteuse sur la séquence d'acides nucléiques de test.

13. Procédé pour détecter un ou plusieurs polymorphisme(s) dans une séquence d'acides nucléiques cible, le procédé comprenant :
la fourniture d'une sonde selon l'une quelconque des revendications 1 à 11 ;
la mise en contact de la sonde avec une séquence d'acides nucléiques de test, la séquence de test comportant une première séquence d'acides nucléiques cible et une seconde séquence d'acides nucléiques cible ;
le fait de permettre que la première séquence d'acides nucléiques de la sonde réalise une hybridation sur la première séquence d'acides nucléiques cible ; et que la seconde séquence d'acides nucléiques de la sonde réalise une hybridation sur la seconde séquence d'acides nucléiques cible ; et
la détermination de la Tm de la première séquence de sonde qui a réalisé une hybridation avec la première séquence cible ; et
la détermination de la Tm de la seconde séquence de sonde qui a réalisé une hybridation avec la seconde séquence cible ;
la comparaison de la Tm déterminée de la première séquence de sonde avec la Tm attendue de la première séquence de sonde ;
la normalisation de la Tm déterminée de la seconde séquence de sonde sur la base de la différence entre les Tm déterminée et attendue de la première séquence de sonde ;
dans lequel la Tm normalisée de la seconde séquence de sonde est indicative de la présence d'une séquence du type sauvage ou d'une séquence variante.
